# NEUE EUROPÄISCHE PATENTSCHRIFT

(11) **EP 0 983 252 B2**
(45) Veröffentlichungstag und Bekanntmachung des Hinweises auf die Entscheidung über den Einspruch: **17.06.2015**
(45) Hinweis auf die Patenterteilung: 17.10.2007
(21) Anmeldenummer: 98925547.6
(22) Anmeldetag: 06.05.1998
(51) Int. Cl.: C07D 301/08

(54) **VERFAHREN UND REAKTOR ZUR HERSTELLUNG VON ETHYLENOXID**
METHOD AND REACTOR FOR PRODUCING ETHYLENE OXIDE
PROCEDE ET REACTEUR POUR LA FABRICATION D'OXYDE D'ETHYLENE

(30) Priorität: 07.05.1997 DE 19719375
(43) Veröffentlichungstag der Anmeldung: 08.03.2000
(73) Patentinhaber: Prosernat, 92042 Paris la Défense Cedex (FR)
(72) Erfinder: HEISEL Michael, 82049 Pullach (DE)
(74) Vertreter: Keller, Günter
(86) Internationale Anmeldenummer: PCT/EP1998/002673
(87) Internationale Veröffentlichungsnummer: WO 1998/050375

(56) Entgegenhaltungen:
- EP-A- 0 081 948
- EP-A- 0 130 807
- EP-A- 0 339 748
- EP-A- 0 529 329
- EP-A- 0 532 325
- EP-A- 0 534 195
- DE-A- 3 339 570
- GB-A- 1 116 345
- GB-A- 2 161 596
- US-A- 4 882 444
- PATENT ABSTRACTS OF JAPAN vol. 095, no. 005, 30.Juni 1995 & JP 07 048305 A (LION CORP.), 21.Februar 1995,

## Beschreibung

Die Erfindung betrifft ein Verfahren zur Herstellung von Ethylenoxid aus einem gasförmigen Einsatz - in einer gekühlten exothermen katalytischen Reaktion von Ethylen und Sauerstoff aus diesem Einsatzgas in parallel durchströmten Reaktionszonen, außerdem einen Reaktor für dieses Verfahren mit Katalysatorpartikeln zwischen gekühlten Trennwänden in mindestens einem Reaktorbehälter.

Aus der Zeitschrift Hydrocarbon Processing, March 1997, Seite 134 ist ein solches Verfahren bekannt. Es wird in Zusammenhang mit einem Röhrenreaktor mit Katalysatorpartikeln in den Rohren verwendet. Die Rohre werden auf der Mantelseite des Reaktors mit siedendem Wasser gekühlt.

Die Aufteilung des Verfahrensstromes und der Katalysatorpartikel auf mehrere Rohre stellt sicher, daß im Falle einer Betriebsstörung eine sich selbst beschleunigende Reaktion hervorgerufen durch örtliche Überhitzung sich auf ein Reaktionsrohr beschränkt und nicht den ganzen Reaktor erfaßt. Diese Verfahrensführung und Reaktorkonstruktion hat jedoch auch mehrere Nachteile.
- Der Reaktormantel muß auf den Kühlmitteldruck ausgelegt sein, in der Praxis ca. 70 bar. Dadurch ist der Mantel sehr dick und damit teuer, schwer zu transportieren und Baustellenmontage ist ausgeschlossen.
- Die Rohrböden sind bei großem Durchmesser sehr dick und damit teuer und durch Wärmespannungen gefährdet.
- Die vielen Reaktionsrohre sind nur mit großem Aufwand in die dicken Rohrböden einzuschweißen.
- Die vielen Reaktionsrohre sind nur mit großem Aufwand zu befüllen. Insbesondere ist auf gleichen Druckverlust in den vielen verschiedenen Rohren zu achten, damit nicht ein wegen hohem Druckabfall zu wenig beaufschlagtes Reaktionsrohr überhitzt wird.
- Wegen des hohen Gewichts wird für den Reaktor C-Stahl verwendet, obwohl Rost damit unvermeidlich ist. Rost wirkt aber als ein Katalysatorgift. Bei Katalysatorentleerung muß deshalb der Reaktor sandgestrahlt werden, was bei der großen Zahl von Reaktionsrohren ein erheblicher zeitlicher und finanzieller Aufwand ist.
- Nur stehende Reaktoren sind realisierbar.
- Die Kühlflache pro Katalysatorvolumen ist nur in engen Grenzen wählbar.

Aufgabe der Erfindung ist daher eine einfache Verfahrensführung im Reaktor und eine einfachere Konstruktion des Reaktors verbunden mit sicherem Betrieb bei Störungen und Vermeidung der genannten Nachteile.

Diese Aufgabe wird erfindungsgemäß gelöst von einem Verfahren mit den Merkmalen des Anspruchs 1 und einem Reaktor mit den Merkmalen des Anspruchs 6. Ausführungen der Erfindung sind Gegenstand von Unteransprüchen.

Kennzeichnend an der Erfindung ist unter anderem, daß beim Verfahren die Reaktionszonen durch gekühlte Trennwände gebildet werden und die Kühlung durch ein innerhalb der Trennwände strömendes Fluid bewerkstelligt wird und daß beim Reaktor die gekühlten Trennwände mit Hilfe von Metallplatten gebildet werden und zur Kühlung in den Metallplatten Hohlräume in Form von Kanälen zur Aufnahme und zum Durchleiten eines Kühlmediums angeordnet sind.

Die durch die gekühlten Trennwände verwirklichten separaten Reaktionszonen ergeben ein sicheres Verfahren auch bei den genannten Betriebsstörungen, da Überhitzung benachbarter Reaktionszonen ausgeschlossen ist. Die Metallplatten sind als kühlbare oder heizbare Panele im Handel erhältlich und ermöglichen kostengünstige Lösungen für die Reaktoreinbauten.

In einer vorteilhaften Ausführung des Verfahrens enthält der gasförmige Einsatz neben den Bestandteilen Ethylen und Sauerstoff auch Stickstoff und/oder Methan. Es kann damit auch mit Stickstoff verunreinigter Sauerstoff oder Luft eingesetzt werden. Mit Methan im Einsatz verbrennt bei zu hohen Betriebstemperaturen vermehrt Methan. Diese Reaktion ist weniger exotherm verglichen mit der Verbrennung von Ethylen, so daß die örtliche Überhitzung verringert wird. Außerdem bleibt der Wertstoff Ethylen erhalten und kann dem Prozeß wieder zugeführt werden.

Vorteilhafterweise wird das Austrittsgas aus den Reaktionszonen mit dem gebildeten Ethylenoxid einer Abtrennung des Ethylenoxid, vorzugsweise durch eine Wasserwäsche, unterzogen und so als Produkt gewonnen.

Ein nach der Abtrennung des Ethylenoxids verbleibendes Restgas kann einer CO₂-Abtrennung, vorzugsweise einer Aminwäsche, zugeführt werden und das weitgehend in der katalytischen Reaktion wiederverwendet werden. Dies führt zu einer guten Ausnutzung des Wertstoffes Ethylen.

Vorzugsweise wird bei der katalytischen Reaktion im erfindungsgemäßen Verfahren eine Raumgeschwindigkeit zwischen 5 000 und 50 000 h⁻¹, bevorzugt zwischen 7 000 und 15 000 h⁻¹ verwendet. Sie kann wegen des niedrigeren Druckabfalls im Reaktor höher gewählt werden als beim Stand der Technik mit meist unter 5 000 h⁻¹. Damit werden drei Vorteile erzielt:
1. Die hohen Raumgeschwindigkeiten führen zu besserer Selektivität des Katalysators und ermöglichen damit einen besseren Ausnutzungsgrad des eingesetzten Ethylen, d.h. die Wirtschaftlichkeit des Verfahrens steigt.
2. Bessere Selektivität bedeutet, daß die unerwünschte Nebenreaktion der vollständigen Verbrennung des Ethylen zu CO₂ und Wasser besser unterdrückt wird. Diese Nebenreaktion ist sehr stark exotherm, d.h. gibt viel Wärme ab. Wo diese Reaktion auftritt steigt folglich die Temperatur. Bei steigender Temperatur wird die Selektivität des Katalysators schlechter, so daß die unerwünschte Nebenreaktion noch verstärkt wird. Das kann soweit gehen, daß Katalysator und/oder Reaktor irreversibel geschädigt werden. Das erfindungsgemäße Verfahren verbessert die Selektivität und erhöht damit die Sicherheit der Produktion von Ethylenoxid.
3. Das Katalysatorvolumen, das für den Umsatz einer bestimmten Menge Rohgas erforderlich ist, wird kleiner. Damit kann der gesamte Reaktor kleiner und folglich billiger werden. Da im Reaktor dann auch weniger von dem reaktionsfähigen Gas enthalten ist, wird auch durch diesen Effekt die Sicherheit weiter erhöht.

Bei dem erfindungsgemäßen Reaktor werden mehrere Metallplatten, vorzugsweise senkrecht, mit Abstand voneinander zu einem Metallplattenpaket zusammengefügt und bilden so einen Freiraum, in den die Katalysatorpartikel geschüttet werden. Rohrböden entfallen und die Reaktionsräume zwischen den Platten sind ähnlich zu befüllen wie ein Festbett ohne Kühlung. Dies ist eine wesentliche Verbesserung gegenüber einem Reaktor nach dem Stand der Technik.

Die Metallplattenpakete werden aus ebenen, vorzugsweise parallel angeordneten Platten gebildet. Solche Platten, auch Plattenpakete, sind kostengünstig im Handel erhältlich.

Bei dem erfindungsgemäßen Reaktor werden mehrere Metallplattenpakete so nebeneinander im Reaktorbehälter angeordnet, daß sie ein Modul aus Plattenpaketen bilden, in dem die Plattenpakete parallel vom Einsatzgas durchströmt werden. Dies ist, insbesondere in liegenden Behältern, leicht zu verwirklichen, ohne an Baugrenzen zu stoßen, und es werden im Verfahren niedrige Druckabfälle ermöglicht.

Es werden mehrere Module im gleichen Reaktorbehälter, vorzugsweise übereinander, angeordnet und nacheinander vom Einsatzgas durchströmt. Zusammen mit der Wahlmöglichkeit zwischen stehenden und liegenden Reaktorbehältern kann der Reaktor so dem verfügbaren Platz und dem zulässigen Druckabfall im Reaktor optimal angepaßt werden.

Weitere Vorteile des erfindungsgemäßen Verfahrens ergeben sich aus dem Zusammenwirken der Merkmale der Erfindung mit denen ihrer günstigen Ausführungsformen:
- Der Reaktormantel muß nur den Gasdruck aushalten, nicht den höheren Druck des Dampfs, der bei der Kühlung erzeugt wird.
- Der Reaktor ist wesentlich leichter als ein Reaktor nach dem Stand der Technik und kann aus Edelstahl gebaut werden, so daß die Probleme von C-Stahl entfallen.
- Die Kühlflache pro Katalysatorvolumen ist in sehr weiten Grenzen frei wählbar.
- Weil die erfindungsgemäßen Reaktoren bei gleicher Leistung wesentlich leichter sind, werden Transport, Montage und Fundamente billiger als bei Reaktoren nach dem Stand der Technik.
- Baubarkeitsgrenzen und Sicherheitsbedenken begrenzen die an einem Standort installierbare Produktionskapazität nicht.

Die Erfindung wird anhand einer Ausführungsform mit einer Figur näher erläutert.

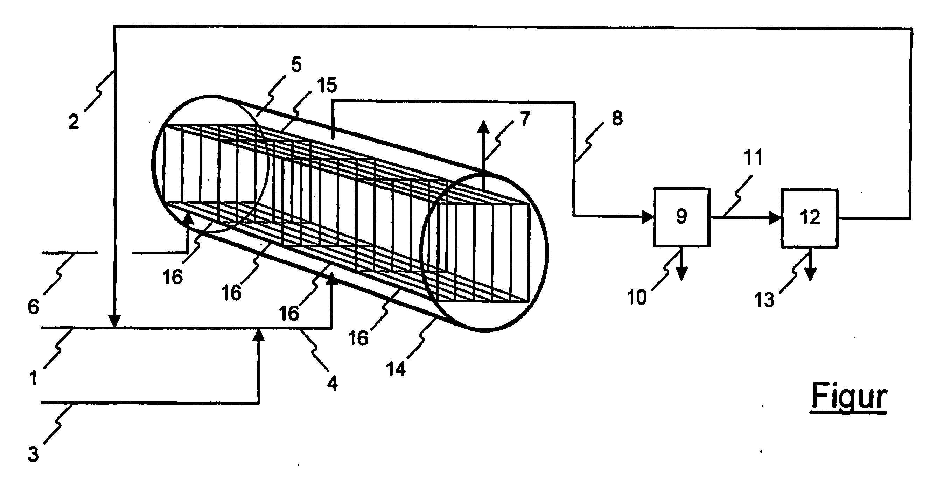

Die Figur zeigt schematisch eine Ausführung des Verfahrens mit einem Reaktor, der ein Modul aufweist.

Ethylen 1 und Recyclegas 2 aus einem hinteren Anlagenteil wird mit Sauerstoff 3 zu einem Einsatz 4 vermischt und einem Reaktor 5 zugeführt, der mit Kesselspeisewasser 6 gekühlt wird, so daß dabei Dampf 7 anfällt. Vom Austrittsgas 8 aus dem Reaktor 5 wird in einer Wasserwäsche 9 Ethylenoxid 10 abgetrennt und das Restgas Reaktor 5 wird in einer Wasserwäsche 9 Ethylenoxid 10 abgetrennt und das Restgas 11 mit dem im Reaktor 5 erzeugten Nebenprodukt CO₂ einer Aminwäsche 12 zugeführt, in der das CO₂ 13 abgetrennt wird. Nach weiteren Reinigungsschritten, die in der Figur nicht dargestellt sind, wird das insbesondere vom CO₂ gereinigte Restgas als Recyclegas 2 ganz oder teilweise im Einsatz 4 des Reaktors wieder verwendet.

Die Figur zeigt außerdem schematisch den Reaktor 5 als liegenden Behälter 14, in dem ein Modul 15 aus Plattenpaketen 16 angeordnet ist. Angedeutet ist, daß die Pakete 16 aus gekühlten parallelen Platten bestehen, deren Kanten in der Figur als Linie dargestellt sind. In ihrem Aufbau können die gekühlten Platten den Heizkörpern für Wohn- und Büroräume ähneln. Die Katalysatorschüttungen zwischen den Platten sind in der Figur nicht dargestellt.

Ein typischer Einsatz für das erfindungsgemäße Verfahren mit dem erfindungsgemäßen Reaktor enthält ungefähr
30 % Ethylen,
60% Methan,
5 % Sauerstoff und
5 % Stickstoff und Wasserdampf.

## Patentansprüche

1. Verfahren zur Herstellung von Ethylenoxid aus einem gasförmigen Einsatz - in einer gekühlten, exothermen, katalytischen Reaktion von Ethylen und Sauerstoff aus diesen Einsatzgasen in parallel durchströmten Reaktionszonen, **dadurch gekennzeichnet, dass** die Reaktionszonen von gekühlten Trennwänden begrenzt werden und die Kühlung durch ein innerhalb der Trennwände strömendes Fluid bewerkstelligt wird, wobei
die gekühlten Trennwände mit Hilfe von Metallplatten gebildet werden und zur Kühlung in den Metallplatten Hohlräume in Form von Kanälen zur Aufnahme und zum Durchleiten eines Kühlmediums angewandt sind, wobei jeweils mehrere Metallplatten senkrecht, mit Abstand voneinander zu einem Metallplattenpaket zusammengefügt werden und so einen Freiraum bilden, in den die Katalysatorpartikel geschüttet werden und die Metallplattenpakete aus ebenen, parallel angeordneten Platten gebildet werden und mehrere Metallplattenpakete so nebeneinander im Reaktorbehälter angeordnet werden, dass sie ein Modul aus Plattenpaketen bilden, in dem die Plattenpakete parallel vom Einsatzgas durchströmt werden, wobei mehrere Module im gleichen Reaktorbehälter vorhanden sind, die vorzugsweise übereinander angeordnet sind, und nacheinander vom Einsatzgas durchströmt werden.

2. Verfahren nach Anspruch 1, **dadurch gekennzeichnet, dass** der gasförmige Einsatz neben den Bestandteilen Ethylen und Sauerstoff auch Stickstoff und/oder Methan enthält.

3. Verfahren nach Anspruch 1 oder 2, **dadurch gekennzeichnet, dass** das Austrittsgas aus den Reaktionszonen mit dem gebildeten Ethylenoxid einer Abtrennung des Ethylenoxids, vorzugsweise durch eine Wasserwäsche, unterzogen wird.

4. Verfahren nach einem der Ansprüche 1 bis 3, **dadurch gekennzeichnet, dass** ein nach der Abtrennung des Ethylenoxids verbleibendes Restgas einer CO₂-Abtrennung, vorzugsweise einer Aminwäsche, zugeführt wird und dass das weitgehend von CO₂ befreite Restgas mindestens teilweise rezykliert, dem Einsatz zugemischt und in der katalytischen Reaktion wiederverwendet wird.

5. Verfahren nach einem der Ansprüche 1 bis 4, **dadurch gekennzeichnet, dass** bei der katalytischen Reaktion eine Raumgeschwindigkeit zwischen 5000 und 50000 h⁻¹, bevorzugt zwischen 7000 und 15000 h⁻¹ verwendet wird.

6. Reaktor zur Herstellung von Ethylenoxid nach einem der Verfahren 1 bis 5 mit Katalysatorpartikeln zwischen gekühlten Trennwänden in mindestens einem Reaktorbehälter, **dadurch gekennzeichnet, dass** die gekühlten Trennwände mit Hilfe von Metallplatten gebildet werden und zur Kühlung in den Metallplatten Hohlräume in Form von Kanälen zur Aufnahme und zum Durchleiten eines Kühlmediums angewandt sind, wobei jeweils mehrere Metallplatten senkrecht, mit Abstand voneinander zu einem Metallplattenpaket zusammengefügt werden und so einen Freiraum bilden, in den die Katalysatorpartikel geschüttet werden und die Metallplattenpakete aus ebenen, parallel angeordneten Platten gebildet werden und mehrere Metallplattenpakete so nebeneinander im Reaktorbehälter angeordnet werden, dass sie ein Modul aus Plattenpaketen bilden, in dem die Plattenpakete parallel vom Einsatzgas durchströmt werden, wobei mehrere Module im gleichen Reaktorbehälter vorhanden sind, die vorzugsweise übereinander angeordnet sind, und nacheinander vom Einsatzgas durchströmt werden.

## Claims

1. Process for the production of ethylene oxide from a gaseous feedstock - in a cooled exothermic, catalytic reaction of ethylene and oxygen from these feed gases in parallel flow-through reaction zones, **characterized in that** the reaction zones are limited by cooled dividing walls and the cooling is achieved by a fluid that flows inside the dividing walls, the cooled dividing walls being formed using metal plates and for cooling in the metal plates cavities in the form of channels for receiving and for passing through a cooling medium being used, wherein in each case several metal plates being joined vertically, with some distance to each other to form a packet of metal plates and thus forming a free space in which the catalyst particles are poured and the metal plate packets being formed from flat, parallel plates and several metal plate packets being arranged beside one another in the reaction vessel, such that they form a module of plate packets in which the feed gas flows in a parallel manner through the plate packets, and several modules being present in the same reaction vessel, preferably arranged on top of each other, with the feed gas flowing through them successively.

2. Process according to claim 1, **characterized in that** the gaseous feedstock also contains nitrogen and/or methane in addition to the components ethylene and oxygen.

3. Process according to claim 1 or 2, **characterized in that** the discharged gas from the reaction zones with the ethylene oxide that has been formed is subjected to a separation of the ethylene oxide, preferably by a water washing.

4. Process according to any one of claims 1 to 3, **characterized in that** a residual gas that remains after the ethylene oxide has been separated is fed to a CO₂ separation, preferably by an amine washing, and **in that** the residual gas from which the CO₂ is largely removed is at least partially recycled, mixed with the feedstock and reused in the catalytic reaction.

5. Process according to any one of claims 1 to 4, **characterized in that** a space velocity of between 5,000 and 50,000 h⁻¹, preferably between 7,000 and 15,000 h⁻¹, is used in the catalytic reaction.

6. Reactor for the production of ethylene oxide according to any one of processes 1 to 5 with catalyst particles between cooled dividing walls in at least one reaction vessel, **characterized in that** the cooled dividing walls are formed using metal plates and for cooling in the metal plates cavities in the form of channels for receiving and for passing through a cooling medium are used, wherein in each case several metal plates being joined vertically, with some distance to each other to form a packet of metal plates and thus forming a free space in which the catalyst particles are poured and the metal plate packets being formed from flat, parallel plates and several metal plate packets being arranged beside one another in the reaction vessel, such that they form a module of plate packets in which the feed gas flows in a parallel manner through the plate packets, and several modules being present in the same reaction vessel, preferably arranged on top of each other, with the feed gas flowing through them successively.

## Revendications

1. Procédé pour la fabrication d'oxyde d'éthylène par une charge sous forme gazeuse d'éthylène et d'oxygène - dans une réaction refroidie, exothermique, catalytique de ces gaz de charge dans des zones de réaction traversées en parallèle, **caractérisé en ce que** les zones de réaction sont délimitées par des parois de séparation refroidies et le refroidissement est réalisé par un fluide circulant à l'intérieur des parois de separation, les parois de séparation refroidies étant formées à l'aide de plaques de métal et, pour le refroidissement dans les plaques de métal, des cavités en forme de canaux étant utilisés pour l'admission et la conduite d'un milieu de refroidissement, à chaque fois plusieures plaques de métal verticales étant unies en un paquet de plaques de métal avec un écart les unes par rapport aux autres ainsi créant un espace libre dans lequel les particules du catalyseur sont versées, et les paquets de plaques de métal étant formés de plaques planes, disposées en parallèle, et plusieurs paquets de plaques de métal étant disposés si bien côte à côte dans le récipient du réacteur qu'ils forment un module de paquets de plaques, les paquets de plaques étant traversés en parallèle par le gaz de charge, et plusieurs modules étant présents dans le même récipient de réacteur, disposés de préférence les uns au-dessus des autres, et traversés successivement par le gaz de charge.

2. Procédé selon la revendication 1, **caractérisé en ce que** la charge sous forme gazeuse comprend outre les composants éthylène et oxygène également de l'azote et/ou du méthane.

3. Procédé selon la revendication 1 ou 2, **caractérisé en ce que** le gaz de sortie des zones de réaction avec l'oxyde d'éthylène formé est soumis à une séparation de l'oxyde d'éthylène, de préférence par un lavage à l'eau.

4. Procédé selon l'une quelconque des revendications 1 à 3, **caractérisé en ce que**, après la séparation de l'oxyde d'éthylène, un gaz résiduel restant est amené à une séparation du CO₂, de préférence à un lavage avec une amine, et **en ce que** le gaz résiduel exempt en grande partie du CO₂ est recyclé au moins en partie, melangé à la charge et réemployé dans la réaction catalytique.

5. Procédé selon l'une quelconque des revendications 1 à 4, **caractérisé en ce qu'**une vitesse spatiale entre 5000 et 50000 h-¹ est utilisée, de préférence entre 7000 et 15000 h⁻¹, lors de la réaction catalytique.

6. Réacteur pour la fabrication d'oxyde d'éthylène selon l'un quelconque des procédés 1 à 5 avec des particules du catalyseur entre parois de séparation refroidies dans au moins un récipient de réacteur, **caractérisé en ce que** les parois de séparation refroidies sont formées à l'aide de plaques de métal et pour le refroidissement dans les plaques de metal, des cavités en forme de canaux sont utilisés pour l'admission et la conduite d'un milieu de refroidissement, à chaque fois plusieures plaques de métal verticales étant unies en un paquet de plaques de métal avec un écart les unes par rapport aux autres ainsi créant un espace libre dans lequel les particules du catalyseur sont versées, et les paquets de plaques de métal étant formés de plaques planes, disposées en parallèle, et plusieurs paquets de plaques de métal étant disposés si bien côte à côte dans le récipient du réacteur qu'ils forment un module de paquets de plaques, les paquets de plaques étant traversés en parallèle par le gaz de charge, et plusieurs modules étant présents dans le même récipient de réacteur, disposés de préférence les uns au-dessus des autres, et traversés successivement par le gaz de charge.
